(19) Europäisches Patentamt
European Patent Office
Office européen des brevets

(11) Numéro de publication: **0 331 576 A2**

# (12) DEMANDE DE BREVET EUROPEEN

(21) Numéro de dépôt: **89400566.9**

(22) Date de dépôt: **01.03.89**

(51) Int. Cl.⁴: **C 07 D 323/00**
C 07 D 407/04, C 07 D 407/06

(30) Priorité: **02.03.88 FR 88002613**

(43) Date de publication de la demande:
**06.09.89 Bulletin 89/36**

(84) Etats contractants désignés:
**AT BE CH DE ES FR GB GR IT LI LU NL SE**

(71) Demandeur: **Etablissement Public dit: CENTRE NATIONAL DE LA RECHERCHE SCIENTIFIQUE (CNRS)**
**15, Quai Anatole France**
**F-75007 Paris (FR)**

(72) Inventeur: **Maillard, Bernard**
**22, rue de Chantilly**
**F-33600 Pessac (FR)**

**Bourgeois, Marie-Josephe**
**13, Allée de la Garennotte**
**F-33610 Cestas (FR)**

**Montaudon, Evelyne**
**132, avenue Pasteur Le Haillan**
**F-33150 St. Medard en Jalles (FR)**

**Lalande, Robert**
**71, rue Loustadot**
**F-33170 Gradignan (FR)**

(74) Mandataire: **Warcoin, Jacques et al**
**Cabinet Régimbeau 26, avenue Kléber**
**F-75116 Paris (FR)**

## (54) Ethers couronnes fonctionnalisés, leur préparation ainsi que leur application en tant qu'agents de complexation d'ions.

(57) La présente invention concerne des éthers couronnes fonctionnalisés, leur procédé de préparation ainsi que leur application en tant qu'agents de complexation d'ions, en particulier de cations.

Les éthers couronnes fonctionnalisés selon l'invention répondent à la formule générale (I) :

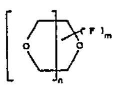

dans laquelle : n = 3,4 ou 5 ; m = 1,2,3 ou 4 ; les groupes F, identiques ou différents, sont choisis parmi :

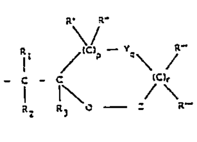

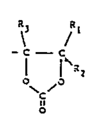

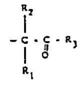

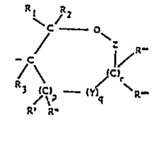

dans lesquelles : $R_1$, $R_2$ et $R_3$ sont choisis parmi H, aryle, cyano, halogéno et les groupes R, -SOR et $SO_2R$ où R représente un groupe $C_{1-10}$ alcoyle, $C_{1-10}$ alcényle ou $C_{1-10}$ alcynyle, lesdits groupes étant linéaires ou ramifiés et pouvant éventuellement être substitués par un ou plusieurs radicaux choisis parmi les radicaux hydroxy, alcoxy, aryloxy, alcoxycarbonyle, carbonyle, halogéno, aryle, nitrile ou un groupe organométallique de formule $-M(X)_3$ dans laquelle M = Si ou Sn et les symboles X, identiques ou différents, sont choisis parmi les radicaux $C_{1-5}$ alcoyle, $C_{1-5}$ alcényle, $C_{1-5}$ alcynyle ou aryle ; R′,R″,R‴ et R⁗, identiques ou différents, sont choisis parmi H, $C_{1-5}$ alcoyle, $C_{1-5}$ alcényle, $C_{1-5}$ alcynyle ou aryle, les radicaux R′ et R″ d'une part et R‴ et R⁗ d autre part, pouvant en outre former ensemble un groupe carbonyle ;

Y est choisi parmi Si, Sn, O, SO, $SO_2$, $-\overset{A}{\overset{|}{N}}-$, avec A choisi parmi H, $C_{1-5}$ alcoyle, $C_{1-5}$ alcényle, $C_{1-5}$ alcynyle ou aryle ; Z représente une liaison directe, Si, Sn, ou encore un groupe $-\underset{O}{\underset{\|}{C}}-$ ou $-O-\underset{O}{\underset{\|}{C}}-$

q = 0 ou 1

$0 \leq p + q + r +$ (nombre d'atomes de la chaîne principale de Z) $\leq 4$.

EP 0 331 576 A2

## Description

# ETHERS COURONNES FONCTIONNALISES, LEUR PREPARATION AINSI QUE LEUR APPLICATION EN TANT QU'AGENTS DE COMPLEXATION D'IONS

La présente invention concerne des éthers couronnes fonctionnalisés, leur procédé de préparation ainsi que leur application en tant qu'agents de complexation d'ions, en particulier de cations, ou encore de catalyseurs de transfert de phase.

Bien que l'intérêt et le nombre des éthers couronnes fonctionnalisés croissent de jour en jour, il n'existe pas de procédé de fonctionnalisation directe des éther couronnes, ce qui impose de sévères limitations dans la nature et le nombre des radicaux de fonctionnalisation que l'on peut envisager.

En effet, dans la technique antérieure, par exemple illustrée par le brevet US n° 4 474 963, il était uniquement connu de fonctionnaliser directement les éthers couronnes lors de leur cyclisation, avec la seule possibilité limitée de procéder à des dérivatisations ultérieures. Il fallait donc envisager au cas par cas, des synthèses particulières qui sont généralement difficiles à mettre en oeuvre, n'autorisent que des fonctionnalisations très restreintes et de surcroît avec de très faibles rendements. Il était donc fort utile de pouvoir disposer d'un procédé de fonctionnalisation directe qui, à partir des éthers couronnes facilement disponibles dans le commerce, puisse conduire dans de bonnes conditions à divers éthers couronnes fonctionnalisés, et en particulier à de nouveaux dérivés.

La présente invention concerne en particulier de nouveaux éthers couronnes fonctionnalisés répondant à la formule générale (1) :

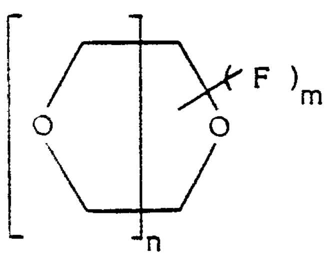

dans laquelle
n = 3,4,5,6,7 ou 8
m = 1,2,3 ou 4
chaque liaison pénétrante dans le cycle, portant un groupe de fonctionnalisation F, peut être attachée à n'importe quel atome de carbone du cycle,
les groupes F, identiques ou différents, sont choisis parmi :

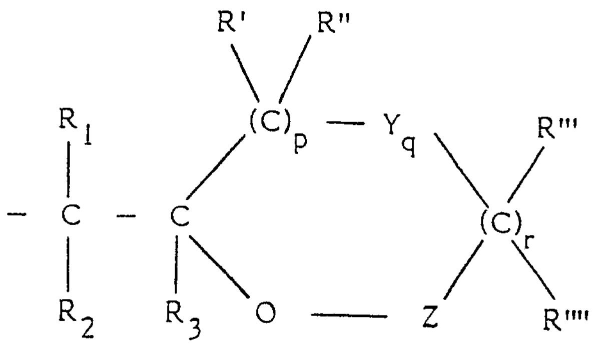

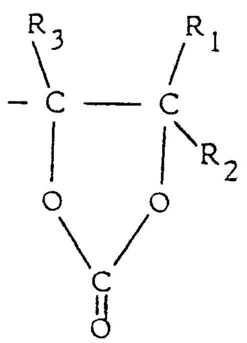

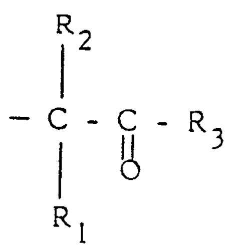

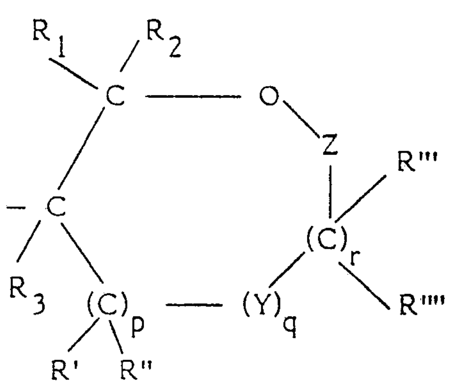

dans lesquelles :
$R_1$, $R_2$ et $R_3$ sont choisis parmi H, aryle, cyano, halogéno et les groupes R, -SOR et $SO_2R$ où R représente un groupe $C_{1-10}$ alcoyle, $C_{1-10}$ alcényle ou $C_{1-10}$ alcynyle, lesdits groupes étant linéaires ou ramifiés et pouvant éventuellement être substitués par un ou plusieurs radicaux choisis parmi les radicaux hydroxy, alcoxy, aryloxy, alcoxycarbonyle, carbonyle, halogéno, aryle, nitrile ou un groupe organométallique de formule $-M(X)_3$ dans laquelle M = Si ou Sn et les symboles X, identiques ou différents, sont choisis parmi les radicaux $C_{1-5}$ alcoyle, $C_{1-5}$ alcényle, $C_{1-5}$ alcynyle ou aryle ;
R′,R″,R‴ et R⁗, identiques ou differents, sont choisis parmi H, $C_{1-5}$ alcoyle, $C_{1-5}$ alcényle, $C_{1-5}$ alcynyle ou aryle, les radicaux R′ et R″ d'une part et R‴ et R⁗ d autre part, pouvant en outre former ensemble un groupe carbonyle ;
Y est choisi parmi Si, Sn, O, SO, $SO_2$,

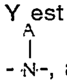, avec A choisi parmi H, $C_{1-5}$ alcoyle, $C_{1-5}$ alcényle, $C_{1-5}$ alcynyle ou aryle ;
Z représente une liaison directe, Si, Sn, ou encore un groupe

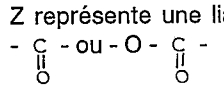

q = 0 ou 1
$O \leqq p + q + r +$ (nombre d'atomes de la chaîne principale de Z) $\leqq 4$.

La présente invention concerne également le procédé de préparation d'un composé de formule générale (1) telle que mentionnée précédemment, qui est caractérisé en ce que l'on fait réagir un éther couronne de formule (Ia) :

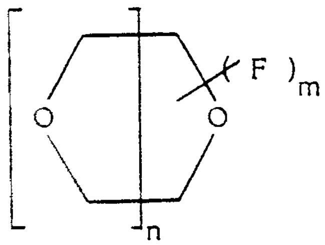

(Ia)

dans laquelle :
m peut prendre les valeurs de 0 à 4,
les autres symboles ayant les significations données précédemment, avec un composé peroxydique insaturé de formule générale (II) :

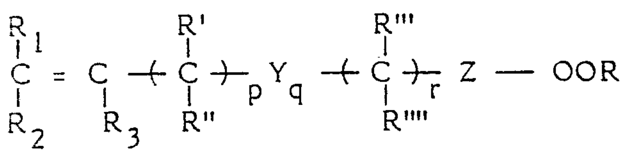

II

dans laquelle :
R représente
. un groupe $C_{1-10}$ alcoyle, $C_{1-10}$ alcényle ou $C_{1-10}$ alcynyle, lesdits groupes étant linéaires ou ramifiés et pouvant eventuellement être substitués par un ou plusieurs radicaux choisis parmi les radicaux hydroxy, alcoxy, aryloxy, alcoxycarbonyle, carbonyle, halogéno, aryle, nitrile ou un groupe organométallique de formule $-M(X)_3$ dans laquelle M = Si ou Sn et les symboles X, identiques ou différents, sont choisis parmi les radicaux $C_{1-5}$ alcoyle, $C_{1-5}$ alcényle, $C_{1-5}$ alcynyle ou aryle ; ou bien
. un groupe acyle de formule
$-\underset{O}{\underset{\|}{C}}-R$ ou $-\underset{O}{\underset{\|}{C}}-OR$
dans lesquelles R a la signification donnée ci-dessus, ou encore
. un groupe organométallique de formule $-M(X)_3$ dans laquelle M = Si ou Sn et les symboles X, identiques ou différents, sont choisis parmi les radicaux $C_{1-5}$ alcoyle, $C_{1-5}$ alcényle, $C_{1-5}$ alcynyle ou aryle ;
$R_1$, $R_2$ et $R_3$, identiques ou différents, sont choisis parmi H, le groupe R défini ci-dessus, aryle, cyano, halogéno et les groupes -SOR et $-SO_2R$ où R a la signification donnée ci-dessus ;
R′,R″,R‴ et R⁗, identiques ou différents, sont choisis parmi H, $C_{1-5}$ alcoyle, $C_{1-5}$ alcényle, $C_{1-5}$ alcynyle ou aryle, les radicaux R′ et R″ d'une part et R‴ et R⁗ d autre part, pouvant en outre former ensemble un groupe carbonyle ;
Y est choisi parmi Si, Sn, O, SO, $SO_2$,
$-\overset{A}{\overset{|}{N}}-$, avec A = H, $C_{1-5}$ alcoyle, $C_{1-5}$ alcényle, $C_{1-5}$ alcynyle ou aryle ;
Z représente une liaison directe, Si, Sn, ou encore un groupe $-\underset{O}{\underset{\|}{C}}-$ ou $-O-\underset{O}{\underset{\|}{C}}-$
q = 0 ou 1
$O \leqq p + q + r +$ (nombre d'atomes de la chaîne principale de Z) $\leqq 4$.

Conformément à la présente invention, il est donc possible d'obtenir des éthers couronnes mono- ou polyfonctionnalisés. Les éthers couronnes fonctionnalisés peuvent en particulier être obtenus dans le cas où les produits de départ sont constitués par des éthers couronnes déjà fonctionnalisés conformément au procédé de l'invention.

Les composés peroxydiques insaturés de formule générale II sont ou bien connus, ou peuvent être aisément préparés par des méthodes en soi connues, par exemple décrites dans "Organic Peroxides D. SWERN (Wiley-Interscience)". A titre d'exemple de ces composés, on citera 3-(t-butylperoxy)propène, le 3-(t-amylperoxy)propène, le 3-cumylperoxy propène, le 3-(t-butylperoxy)2-méthyl propène, le 3-(t-butylpero-

xy)3,3-diméthyl propène, le monoperoxycarbonate de O-isopropényl O,O-t-butyle, le monoperoxycarbonate de O-allyl O,O-t-butyle, le monoperoxycarbonate de O-isopropényl O,O-cumyle, le monoperoxycarbonate de O-méthallyl O,O-t-butyle.

Le procédé de préparation selon l'invention s'analyse en une addition radicalaire de l'éther couronne aux composés peroxydiques précédemment définis. Il faut donc initier la réaction par un processus radicalaire, c'est-à-dire qu'il faut provoquer l'arrachement d'un atome d'hydrogène porté par un carbone en alpha de l'éther couronne. Cet arrachement peut être obtenu par différents processus radicalaires classiques bien connus de l'homme de l'art, par exemple décrits dans l'ouvrage "Free-radical Chemistry Structure and Mechanism - D.C. NONHEBEL and J.C. WALTON (1974)".

A titre d'exemples particuliers de modes d'initiation radicalaire, on citera les décompositions thermique et photolytique d'amorceurs, la photosensibilisation, la radiolyse ou la décomposition catalysée par des ions métalliques, voire encore utilisation d'un système redox.

A titre d'exemples particuliers d'amorceurs, on citera le peracétate de tert.butyle, le perdicarbonate de diéthyle, le peroxyde de benzoyle, le percarbonate d'isopropényle et de tert.butyle, et le peroxyde d'allyle et de tert.butyle, le peroxydicarbonate de di-n-butyle, le peroxydicarbonate de (4-tert-butylcyclohexyl), le peroxydicarbonate de di-sec-butyle, le peroxydicarbonate de bis (2-éthylhexyl), le peroxydicarbonate de dimyristyle, le peroxydicarbonate de dicétyle, le peroxyneodécanoate de t-amyle, le peroxyneodécanoate de tert-butyle, le peroxypivalate de tert-amyle, le peroxypivalate de t-butyle, le peroxyde de bis (3,5,5-triméthyl hexanoyl), le peroxyde de bis (2-méthylbenzoyl), le peroxyde de didécanoyle, le peroxyde de di-octanoyle, le peroxyde de dilauroyle, le peroxydiéthylacétate de tert-butyle, le peroxy-2-éthylhexanoate de tert-butyle, le peroxyde de dibenzoyle, le peroxy isobutyrate de tert-butyle, le 1,1-bis -tert-butyl-peroxy), le 3,3,5-triméthylcyclohexane, le 1,1-bis (tert-butylperoxy)cyclohexane, le peroxy-3,5,5-triméthylhexanoate de tert-butyle, le peroxyisopropylcarbonate de tert-butyle, le 2,2-bis (tert-butylperoxy)butane, le peroxy stéarylcarbonate de tert-butyle et le peroxyacétate de tert-butyle.

On notera à ce sujet que la température de la réaction de fonctionnalisation proprement dite n'est pas critique, mais peut varier principalement en fonction de la nature de l'amorceur utilisé.

Conformément à une autre caractéristique de la présente invention, le rapport des concentrations molaires de l'éther couronne de départ et du composé peroxydique insaturé est compris entre 0,33 et 10. Le rapport particulier retenu, tel que cela sera illustré par les exemples ci-après, aura bien sûr une incidence sur le degré de fonctionnalisation.

On notera enfin que l'éther couronne fonctionnalisé ainsi obtenu sera séparé du milieu réactionnel par tout moyen approprié, en particulier par distillation.

Les éthers couronnes fonctionnalisés, objet de la présente invention, peuvent être utilisés tout d'abord comme intermédiaire de synthèse pour la préparation d'autres éthers couronnes à fonctionnalité très particulière. Ils peuvent également être utilisés en tant que tels, en tant qu'agents de complexation des ions, et en particulier des cations. Enfin, en fonction de la nature particulière du ou des radicaux de fonctionnalisation, les éthers couronnes, objet de la présente invention, peuvent être polymérisés entre eux par exemple par polycondensation. Ils peuvent également être soumis à une réaction de réticulation, en particulier dans le cas où chaque éther couronne comporte plusieurs radicaux de fonctionnalisation.

Enfin, les éthers couronnes fonctionnalisés selon l'invention peuvent également être greffés sur un support par exemple un polystyrène, une silice ou tout autre support habituellement utilisé tel que les résines de charge de colonne de séparation.

D'autres caractéristiques et avantages de la présente invention ressortiront à la lecture des exemples particuliers donnés ci-après à simple titre d'illustration non limitatif.

**Mode opératoire général**

(Les conditions et résultats particuliers sont indiqués dans les schémas et tableaux figurant plus loin dans la description).

Le mélange réactionnel, constitué par un éther couronne et un dérivé peroxydique insaturé tel que défini précédemment, est chauffé sous atmosphère d'azote, dans un réacteur équipé d'un condenseur. Le tableau III ci-après précise la durée de la réaction t exprimée en heure et la température de réaction T exprimée en °C. L'éther couronne de départ en excès est alors éliminé par distillation, puis à partir du résidu, par nouvelle distillation, on sépare l'éther couronne substitué. Si nécessaire, il est possible de procéder à une purification complémentaire, par exemple par chromatographie liquide-solide ($SiO_2$ ; solvant : diéthyléther-acétone). La pureté des éthers couronnes ainsi obtenus est vérifiée par chromatographie en phase gazeuse (3% OV-1/colonne WHP 80/100 Mesh-Intersmat® ICG 112 F).

Dans chaque cas, il a également été procédé à l'analyse centésimale des produits obtenus. On observera que le pourcentage de carbones était généralement inférieur de l'ordre de 1,5% au pourcentage théorique calculé et celui d'hydrogène supérieur de l'ordre de 1%. Ces résultats doivent certainement être attribués à une présence d'eau (<5% poids/poids).

D'autres caractéristiques physiques sont également indiquées dans les tableaux figurant ci-après, en particulier les résultats d'analyse de spectrométrie de masse ainsi que de spectre RMN.

Le choix des conditions particulières de réaction est principalement lié à la nature du composé peroxydique insaturé utilisé. En particulier, les conditions thermiques doivent être choisies de manière telle que ce dérivé peroxydique insaturé subit une décomposition spontanée la plus faible possible, de manière à ce que sa

disparition s'effectue par un processus d'addition-réarrangement homolytique, seul processus permettant la fonctionnalisation du substrat. C'est ainsi qu'une température inférieure ou égale à environ 110°C est généralement choisie pour les peroxydes et une température inférieure ou égale à environ 80°C pour les peresters.

Une fois ce choix effectué la sélection de l'amorceur annexe sera effectuée de tel le sorte qu'à la température de réaction, la décomposition d'environ 99% de ce dernier devra s'effectuer dans un temps raisonnable qui correspondra au temps de réaction.

Schema 1 : fonctionnalisation homolytique d'éthers couronnes

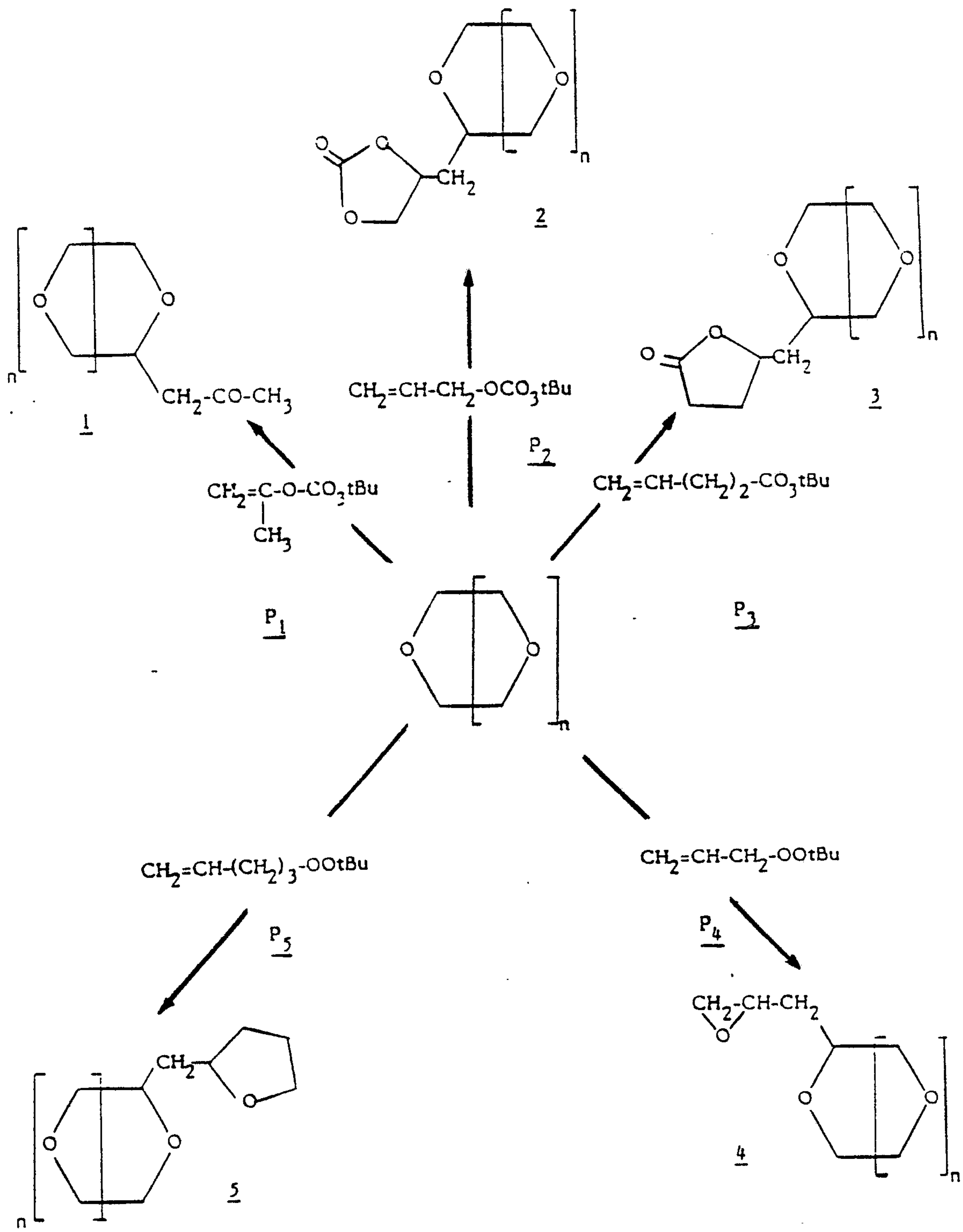


1, 2, 3, 4, 5

a : n = 3

b : n = 4

c : n = 5

TABLEAU I : Dérivés de 12-$O_4$ éther couronne

| Composé | Rendement % (a) | T éb. °C/Torr | $n_D^{20}$ | $^1$H-RMN ($CCl_4$/TMS) (ppm) (c) | $^{13}$C-RMN ($CDCl_3$/TMS) (ppm) (d) |
|---|---|---|---|---|---|
| 1a<br>12-$O_4$-$\overset{3}{C}H_2$-$\overset{2}{C}O$-$\overset{1}{C}H_3$ | 45 | 115/0,1 | 1,4710 | 2,1,s,3H($H_1$); 2,4-2,6,m, 2H($H_3$) ; 3,4-4,1,m, 15H (couronne) | $C_1$:31,0 ; $C_3$:45,8 ; $CH_2$ (couronne): 70,0-70,4-70,7-70,9-73,1 ; CH(couronne): 75,2 ; $C_2$:207,1 |
| 2a<br>12-$O_4$-$\overset{4}{C}H_2$- 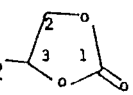 | 30 | 170-180/0,01 (b) | 1,4850 | 3,3-5,m | $C_4$:35,4-36,6 ; $C_2$ et $CH_2$ (couronne): 69,2-69,8-70,0-70,3-70,5-70,8-72,6-72,9 ; $C_3$ et CH (couronne): 74,6-75,0-75,2-75,5 ; $C_1$ : 155,2 |
| 3a<br>12-$O_4$-$\overset{5}{C}H_2$- 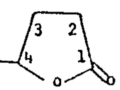 | 38 | 170-180/0,01 (b) | 1,4860 | 1,5-2,7,m,6H($H_5$,$H_3$,$H_2$); 3,5-4,9,m,16H (couronne, $H_4$) | $C_2$,$C_3$:28,2-28,6; $C_5$:37,4-38,5; $CH_2$ (couronne):69,4-70,3-70,6-71,4-73,0-73,5; $C_4$ et CH (couronne):75,9-76-77,8; $C_1$:177,0 |

TABLEAU I (suite)

| Composé | Rendement % (a) | T éb. °C/Torr | $n_D^{20}$ | $^1$H-RMN ($CCl_4$/TMS) (ppm) (c) | $^{13}$C-RMN ($CDCl_3$/TMS) (ppm) (d) |
|---|---|---|---|---|---|
| 4a 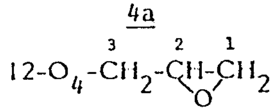 | 60 | 110/0,05 | 1,4730 | 1,2-1,75,m, 2H($H_3$); 2,2-3,m, 3H($H_1$,$H_2$); 3,3-4, 15H(couronne) | $C_3$:34,8-35,5; $C_1$:46,8-47,5; $C_2$:49,3-49,6; $CH_2$(couronne):69,7-70,3-70,4-70,7-70,8-71,0-71,1-73,5-74; CH(couronne): 77,4 |
| 5a 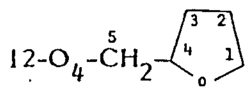 | 55 | 150/0,01 | 1,4775 | 1,15-2,2,m,6H($H_2$,$H_3$,$H_5$); 3,25-4,3,m,18H(couronne, $H_1$,$H_4$) | $C_2$,$C_3$:25,6-25,7-31,74-31,85; $C_5$:37,5-38,4; $C_1$:67,5-67,6 ; $CH_2$ (couronne): 69,6-70,3-70,4-70,5-70,6-70,8-70,9-71-73,5-74,5 ; $C_1$ et CH (couronne):75,8-76,1-77,1-77,3 |

(a) calculé relativement au dérivé peroxydique insaturé

(b) distillation au tube à boules

(c) spectromètre Bruker WP 60 CW. Les protons $H_x$ sont liés au carbone numéroté X.

(d) spectromètre Bruker WP 90 (22,63 MHz) (bande large $^1$H découplé)

TABLEAU II : 2,3-époxy propanation et acétonylation d'éthers couronnes 15-$O_5$ et 18-$O_6$

| Composé | Rendement % (a) | T éb. °C/Torr | $n_D^{20}$ | $^1$H-RMN ($CCl_4$/TMS) (ppm) (c) | $^{13}$C-RMN ($CDCl_3$/TMS) (ppm) (d) |
|---|---|---|---|---|---|
| 1b<br>$15\text{-}O_5\text{-}\overset{3}{C}H_2\text{-}\overset{2}{C}O\text{-}\overset{1}{C}H_3$ | 47 | 120-125/0,01 | 1,4720 | 2,1,s, 3H($H_1$); 2,5-2,8,m, 2H($H_3$); 3,4-4,1,m, 19H (couronne) | $C_1$:30,6; $C_3$:46,0; $CH_2$(couronne):70,0-70,1-70,3-70,6-70,8-72,7; CH(couronne): 75,2; $C_2$:206,9 |
| 1c<br>$18\text{-}O_6\text{-}\overset{3}{C}H_2\text{-}\overset{2}{C}O\text{-}\overset{1}{C}H_3$ | 30 | 155/0,001 (b) | 1,4720 | 2,1,s, 3H($H_1$); 2,4-2,7,m, 2H($H_3$); 3,4-4,1,m,23H (couronne) | $C_1$:30,9; $C_3$:45,9; $CH_2$(couronne): 69,6-70,5-70,8-73; CH(couronne):75,1; $C_2$:207,3 |
| 4b<br>$15\text{-}O_5\text{-}\overset{3}{C}H_2\text{-}\overset{2}{C}H\text{-}\overset{1}{C}H_2$<br>O | 55 | 125-130/0,1 | 1,4750 | 1,3-1,8,m, 2H($H_3$); 2,25-3,1,m, 3H($H_1$,$H_2$); 3,25-4,m, 19H (couronne) | $C_3$:34,8-35,5; $C_1$:46,5-47,2; $C_2$:49,2-49,4; $CH_2$(couronne):69,5-70,1-70,3-70,5-70,7-70,8-70,9-73,2-73,6; CH(couronne): 77,2 |
| 4c<br>$18\text{-}O_6\text{-}\overset{3}{C}H_2\text{-}\overset{2}{C}H\text{-}\overset{1}{C}H_2$<br>O | 50 | 155/0,001 (b) | 1,4752 | 1,3-1,9,m, 2H($H_3$); 2,15-3,1,m, 3H($H_1$,$H_2$); 3,3-4,1,m, 23H (couronne) | $C_3$:34,35-35,0; $C_1$:46,3-46,9; $C_2$:48,9-49,1; $CH_2$(couronne):68,9-69,4-70,2-70,4-73,2-73,6; CH(couronne:76,5 |

(a) calculé relativement au dérivé peroxydique insaturé
(b) distillation au tube à boules
(c) spectromètre Bruker WP 60 CW. Les protons $H_x$ sont liés au carbone numéroté X.
(d) spectromètre Bruker WP 90 (22,63 MHz) (bande large $^1$H découplé)

<u>TABLEAU III</u> : Conditions des réactions de fonctionnalisation

| Dérivé peroxydique insaturé $\underline{P_x}$ | (a) | Initiateur | Rapport molaire $\underline{P_x}$/Initiateur | T°C | t |
|---|---|---|---|---|---|
| $CH_2=C(CH_3)-OCO_3tBu$ | $\underline{P_1}$ | perdicarbonate de diéthyle<br>ou peroxyde de benzoyle | 1 - 0,2<br>1 - 0,1 | 60<br>80 | 12 h<br>24 h |
| $CH_2=CH-CH_2-OCO_3tBu$ | $\underline{P_2}$ | peroxyde de benzoyle | 1 - 0,1 | 80 | 24 h |
| $CH_2=CH-(CH_2)_2-CO_3tBu$ | $\underline{P_3}$ | peroxyde de benzoyle | 1 - 0,1 | 80 | 24 h |
| $CH_2=CH-CH_2-OOtBu$ | $\underline{P_4}$ | peracétate de t-butyle | 1 - 0,1 | 110 | 12 h |
| $CH_2=CH-(CH_2)_3-OOtBu$ | $\underline{P_5}$ | peracétate de t-butyle | 1 - 0,5 | 110 | 12 h |

(a) Rapport : éther couronne/dérivés peroxydique insaturés = 10

TABLEAU IV

Analyse élémentaire

| Composé | C calculé | H calculé | C trouvé | H trouvé |
|---|---|---|---|---|
| 1a | 56,88 | 8,68 | 55,71 | 8,64 |
| 2a | 52,17 | 7,30 | 52,39 | 7,37 |
| 3a | 56,92 | 8,08 | 55,42 | 8,18 |
| 4a | 56,88 | 8,68 | 55,13 | 8,71 |
| 5a | 59,98 | 9,29 | 57,13 | 9,04 |
| 1b | 56,50 | 8,75 | 55,49 | 8,74 |
| 1c | 56,23 | 8,81 | 55,07 | 8,97 |
| 4b | 56,50 | 8,75 | 55,17 | 8,80 |
| 4c | 56,23 | 8,81 | 54,60 | 8,86 |

On indiquera en outre ci-après deux exemples supplémentaires illustrant la pluri-fonctionnalisation d'éthers couronnes.

**1. Polyépoxydation**

Une addition lente du mélange peroxydique à l'éther couronne donnant de meilleurs résultats que le chauffage direct des trois composés, on décrira ci-après ce mode opératoire.

L'éther couronne ($10^{-2}$ mole) est placé dans un ballon surmonté d'un réfrigérant et protégé par un desséchant ; ce ballon est muni d'une tubulure latérale par laquelle arrive l'aiguille d'une seringue à travers un septum. Le ballon est placé dans un bain d'huile régulé à 110° C à l'aide d'un agitateur magnétique chauffant. Un barreau aimanté placé dans le ballon réalise l'homogénéité du milieu durant l'addition du mélange :

peroxyde et d'allyle et de t.butyle ($2.10^{-2}$ mole)

et

peracétate de t.butyle ($10^{-3}$ mole).

Cette addition se fait à l'aide d'un pousse-seringue ayant un débit de 0,66 ml/heure (seringue de 10 ml). Après la fin de l'addition on maintient le mélange à la même température 12 h de plus.

Une simple distillation au tube à boules permet de séparer l'éther couronne monoépoxydé, puis le diépoxydé et le triépoxydé.

**2. Acétonylation d'un éther-couronne monoépoxydé**

Dans un ballon, surmonté d'un réfrigérant et protégé par un desséchant, on place l'éther-couronne monoépoxydé ($2.10^{-2}$ mole), le percarbonate d'isopropényle et de t.butyle ($1.10^{-2}$ mole) et le perdicarbonate de diéthyle ($2.10^{-3}$ mole). Le ballon est plongé dans un bain à 60° C et maintenu à cette température pendant 12 heures.

Par distillation fractionnée on récupère l'éther couronne monoépoxydé qui n'a pas réagi puis l'éther couronne difonctionnel.

On indiquera enfin dans le tableau V ci-après les rendements en dérivés mono- di- ou triépoxydés en fonction du rapport molaire entre l'éther couronne de départ et le dérivé peroxydique insaturé.

TABLEAU V

Rendement par rapport à l'éther couronne de départ consommé (mole %)

| Rapport molaire Couronne | peroxyde | peracétate de t.butyle | Monoépoxyde | Diépoxyde | Triépoxyde | Rendement global par rapport au peroxyde d'allyle |
|---|---|---|---|---|---|---|
| 12-$O_4$ 2 | 1 | 0,1 | 50 | 10 | - | 58 |
| 12-$O_4$ Monoépoxyde 2 | 1 | 0,1 | - | 60 | 3 | 51 |
| 12-$O_4$ 1 | 2 | 0,1 | 23 | 15 | 5 | 33 |
| 12-$O_4$ 1 | 2 (addition en 5 h) | 0,1 (addition en 5 h) | 30 | 23 | 10 | 47 |
| 12-$O_4$ 1 | 3 (addition en 7 h) | 0,1 (addition en 7 h) | 18 | 22 | 14 | 32 |

## Revendications

1/ Ethers couronnes fonctionnalisés répondant à la formule générale (I) :

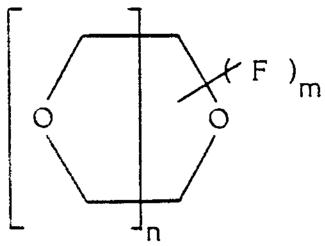

dans laquelle
n = 3,4,5,6,7 ou 8
m = 1,2,3 ou 4
chaque liaison pénétrante dans le cycle, portant un groupe de fonctionnalisation F, peut être attachée à n'importe quel atome de carbone du cycle,
les groupes F, identiques ou différents, sont choisis parmi :

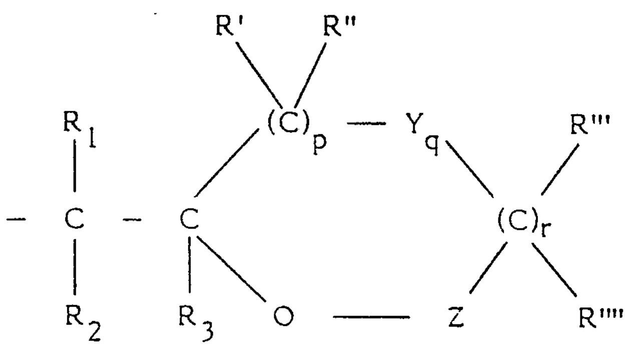

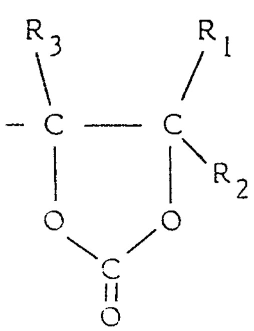

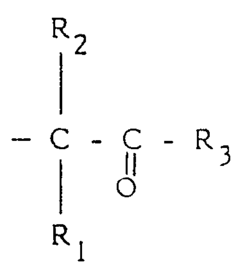

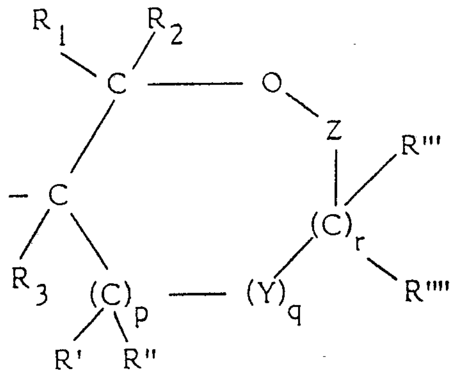

dans lesquelles :
$R_1$, $R_2$ et $R_3$ sont choisis parmi H, aryle, cyano, halogéno et les groupes R, -SOR et $SO_2R$ où R représente un groupe $C_{1-10}$ alcoyle, $C_{1-10}$ alcényle ou $C_{1-10}$ alcynyle, lesdits groupes étant linéaires ou ramifiés et pouvant éventuellement être substitués par un ou plusieurs radicaux choisis parmi les radicaux hydroxy, alcoxy, aryloxy, alcoxycarbonyle, carbonyle, halogéno, aryle, nitrile ou un groupe organométallique de formule $-M(X)_3$ dans laquelle M = Si ou Sn et les symboles X, identiques ou différents, sont choisis parmi les radicaux $C_{1-5}$ alcoyle, $C_{1-5}$ alcényle, $C_{1-5}$ alcynyle ou aryle ;
R′,R″,R‴ et R⁗, identiques ou differents, sont choisis parmi H, $C_{1-5}$ alcoyle, $C_{1-5}$ alcényle, $C_{1-5}$ alcynyle ou aryle, les radicaux R′ et R″ d'une part et R‴ et R⁗ d autre part, pouvant en outre former ensemble un groupe carbonyle ;
Y est choisi parmi Si, Sn, O, SO, $SO_2$,
$-\overset{A}{\overset{|}{N}}-$, avec A choisi parmi H, $C_{1-5}$ alcoyle, $C_{1-5}$ alcényle, $C_{1-5}$ alcynyle ou aryle ;
Z représente une liaison directe, Si, Sn, ou encore un groupe
$-\underset{\underset{O}{\|}}{C}-$ ou $-O-\underset{\underset{O}{\|}}{C}-$
q = 0 ou 1
$O \leqq p + q + r +$ (nombre d'atomes de la chaîne principale de Z) $\leqq 4$.

2/ Ethers couronnes fonctionnalisés selon la revendication 1, répondant à la formule générale :

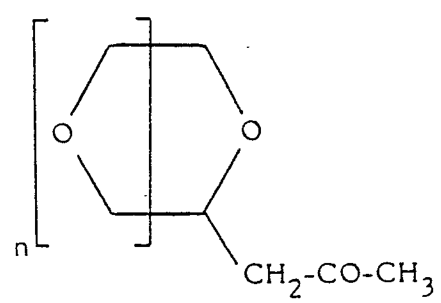

dans laquelle : n = 3, 4 ou 5.

3/ Ethers couronnes fonctionnalisés selon la revendication 1, répondant à la formule générale :

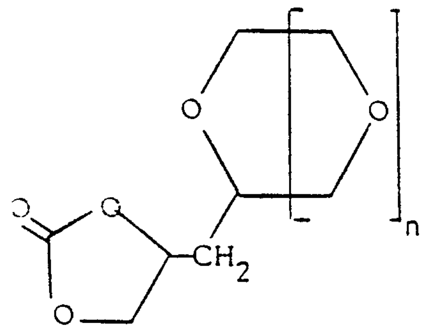

dans laquelle : n = 3, 4 ou 5.

4/ Ethers couronnes fonctionnalisés selon la revendication 1, répondant à la formule générale :

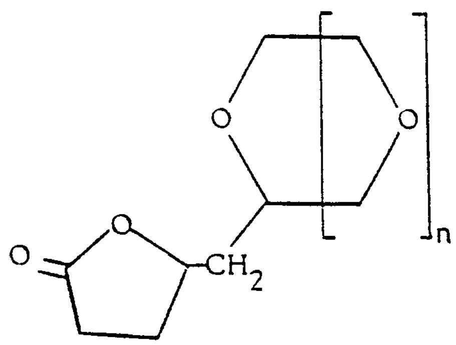

dans laquelle : n = 3,4 ou 5.

5/ Ethers couronnes fonctionnalisés selon la revendication 1, répondant à la formule générale :

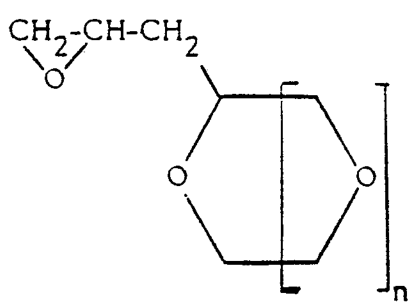

dans laquelle : n = 3,4 ou 5.

6/ Ethers couronnes fonctionnalisés selon la revendication 1, répondant à la formule générale :

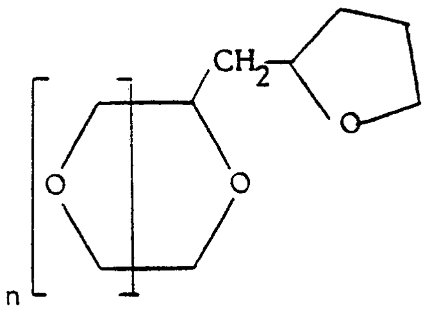

dans laquelle n = 3, 4 ou 5.

7/ Procédé de préparation d'un composé de formule générale (I) tel que mentionné à la revendication 1, caractérisé en ce que l'on fait réagir un éther couronne de formule (Ia) :

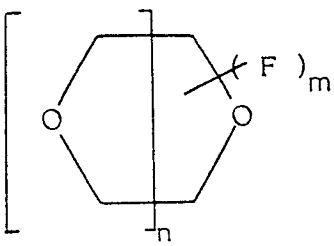

(Ia)

dans laquelle :
m peut prendre les valeurs de 0 à 4,
les autres symboles ayant les significations données à la revendication 1,
avec un composé peroxydique insaturé de formule générale (II) :

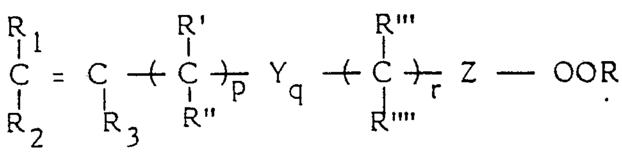

II

dans laquelle :
R représente
. un groupe $C_{1-10}$ alcoyle, $C_{1-10}$ alcényle ou $C_{1-10}$ alcynyle, lesdits groupes étant linéaires ou ramifiés et pouvant eventuellement être substitués par un ou plusieurs radicaux choisis parmi les radicaux hydroxy, alcoxy, aryloxy, alcoxycarbonyle, carbonyle, halogéno, aryle, nitrile ou un groupe organométallique de formule $-M(X)_3$ dans laquelle M = Si ou -Sn et les symboles X, identiques ou différents, sont choisis parmi les radicaux $C_{1-5}$ alcoyle, $C_{1-5}$ alcényle, $C_{1-5}$ alcynyle ou aryle ; ou bien
. un groupe acyle de formule

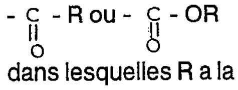

dans lesquelles R a la signification donnée ci-dessus.
ou encore
un groupe organométallique de formule $-M(X)_3$ dans laquelle M = Si ou Sn et les symboles X, identiques ou différents, sont choisis parmi les radicaux $C_{1-5}$ alcoyle, $C_{1-5}$ alcényl, $C_{1-5}$ alcynyle ou aryle ;
$R_1$, $R_2$ et $R_3$, identiques ou différents, sont choisis parmi H, le groupe R défini ci-dessus, aryle, cyano, halogéno et les groupes -SOR et $-SO_2R$ où R a la signification donnée ci-dessus ;
R′, R″, R‴, et R⁗, identiques ou différents, sont choisis parmi H, $C_{1-5}$ alcoyle, $C_{1-5}$ alcényle, $C_{1-5}$ alcynyle ou aryle, les radicaux R′ et R″ d'une part et R‴ et R⁗ d'autre part, pouvant en outre former ensemble un groupe carbonyle ;
Y est choisi parmi Si, Sn, O, SO, $SO_2$,

$-\overset{\overset{A}{|}}{N}-$, avec A = H, $C_{1-5}$ alcoyle, $C_{1-5}$ alcényle, $C_{1-5}$ alcynyle ou aryle ;
Z représente une liaison directe, Si, Sn, ou encore un groupe $-\underset{\underset{O}{\|}}{C}-$ ou $-O-\underset{\underset{O}{\|}}{C}-$

q = 0 ou 1
$O \leqq p + q + r +$ (nombre d'atomes de la chaîne principale de Z) $\leqq 4$.

8/ Procédé selon la revendication 7, caractérisé en ce que la réaction est initiée par un processus radicalaire.

9/ Procédé selon l'une des revendications 7 et 8, caractérisé en ce que l'on ajoute au milieu réactionnel un amorceur, notamment choisi parmi le peracétate de tert.butyle, le perdicarbonate de diéthyle, le peroxyde de benzoyle, le percarbonate d'isopropényle et de tert.butyle, le peroxyde d'allyle et de tert.butyle.

10/ Procédé selon l'une des revendications 7 à 9, caractérisé en ce que le rapport des concentrations molaires d'éthers couronnes de départ et de composés peroxydiques insaturés est compris entre 0,33 et 10.

11/ Procédé selon l'une des revendications 7 à 10, caractérisé en ce que l'éther couronne fonctionnalisé est séparé du milieu réactionnel par distillation.

12/ Application des composés de formule générale (I) selon la revendication 1, en tant qu'agents de complexation d' ions, en particulier le cation, ou bien en tant que catalyseur de transfert de phase.